(19) Europäisches Patentamt
European Patent Office
Office européen des brevets

(11) EP 2 174 643 A1

(12) **EUROPEAN PATENT APPLICATION**

(43) Date of publication:
**14.04.2010 Bulletin 2010/15**

(21) Application number: **08305675.4**

(22) Date of filing: **13.10.2008**

(51) Int Cl.:
***A61K 8/35*** *(2006.01)* ***A61K 8/67*** *(2006.01)*
***A61Q 19/04*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Johnson & Johnson Consumer France SAS**
**92130 Issy-les-Moulineaux (FR)**

(72) Inventors:
- **Gohier, Annie**
  **27310, Trinite De Thouberville (FR)**
- **Joetzjer, Pascale**
  **27400, Louviers (FR)**
- **Marchelidon, Hervé**
  **27340, Tostes (FR)**

(74) Representative: **Warcoin, Jacques et al**
**Cabinet Régimbeau**
**20, rue de Chazelles**
**75847 Paris Cedex 17 (FR)**

(54) **Self tanning compositions containing dihydroxyacetone, a retinoid and ascorbic acid glucoside as a stabilizer**

(57) The invention concerns an emulsion comprising dihydroxyacetone, a retinoid and a glycosylated ascorbic acid.

EP 2 174 643 A1

## Description

Because of the risks associated with sun tanning such as sunburn, many people use self-tanning compositions as a means to either achieve a tan without exposure to the sun, obtain a deeper tan with less exposure to the sun, or to extend the natural life of their suntan.

Products that are currently used for sunless tanning of the skin are based on the reaction of an active chemical present in the product with the skin's amino acids. Such chemicals are well known and include compounds having an aldehyde group, or compounds having a ketone group, such as, for example, dihydroxyacetone (DHA). When applied, DHA interacts with dead skin cells located in the stratum corneum of the epidermis. This interaction causes a color change thereby providing a darkening or tanning effect which typically occurs about 2 to 4 hours after application. However, the color change typically lasts less than five days from the initial application, and it should be interesting to provide a tanning composition which has longer effects.

Retinoids constitute a large group of synthetic and naturally occurring compounds related to retinal, the vitamin A alcohol. In mammals, retinoids fulfill essential roles, including maintenance of epithelial cells. Thus, application of retinoids permits to combat in particular cutaneous aging and certain skin disorders, such as acne or disorders of keratinization or of healing. Moreover, retinoids increase cell proliferation, thus permitting an increase in cell regeneration. Retinol can be useful to remove hyperpigmentation spots and can thus be used to unify skin tone.

It thus would be interesting to combine the tanning properties of DHA and the above-mentioned advantages of retinoids in order to provide an even colour and longer tanning benefit.

Unfortunately, dihydroxyacetone and retinoids, are known to be unstable and are sensitive to external factors such as light or heat. This instability goes against the desired effectiveness and can, moreover, be a source of annoyance to the user, for example when the instability of the active ingredient leads to modifications in the color and/or smell of the composition containing it.

Moreover, it has been shown that DHA and retinoids are incompatible since DHA is more stable at a pH below 5, whereas retinoids, and particularly retinol, show a better stability at a pH above 5. Thus, it is admitted that these two compounds are incompatible in term of pH.

Surprisingly, the inventors have found that by using a glycosylated ascorbic acid, the association of DHA and retinoids becomes possible at a pH ranging from 4 to 5, preferably from 4.1 to 4.8.

The international patent application WO 02/072039 refers to topical preparations and food preparations which can optionally comprise glucoside ascorbate as a whitening agent, retinol and its derivative or dihydroxyacetone. However, the preparation disclosed necessarily includes pyridoxine-$\alpha$-D-glucoside. Moreover, the compounds of the present invention are present among several other agents, it is never stated that the three compounds of the invention are included in the same preparation, and glucoside ascorbate is not disclosed as a stabilizer.

The international patent application WO 2006/009987 relates to a dissolvable film composition for application to the skin which comprises a labile active. Among the labile agents, ascorbic acid, retinoids and dihydroxyacetone are recited. Ascorbyl glucoside is cited among the whitening agents. Moreover, it is never stated that the three compounds are specifically included in the same preparation and that ascorbyl glucoside can be used as a stabilizer.

Therefore the present invention concerns an emulsion comprising: a) dihydroxyacetone, b) a retinoid, and c) a glycosylated ascorbic acid.

Preferably, the emulsion is a stable emulsion.

Preferably, the emulsion according to the invention is a cosmetic emulsion.

More preferably, the emulsion according to the present invention is an emulsion for coloring the skin.

In preferred embodiment, the emulsion is intended to enhance the radiance of the complexion of the skin, in particular of the face, so as to give a healthy appearance.

In another preferred embodiment, the emulsion is a tanning emulsion.

In a further embodiment, the emulsion is an anti-aging emulsion.

Preferably, the emulsion according to the invention does not comprise pyridoxine-$\alpha$-D-glucoside.

By "stable", it should be understood in the sense of the present invention that the emulsion according to the present invention is physically and chemically stable, i.e. that there is no visible phase separation and that all the compounds are chemically stable, i.e. their content is preserved in storage conditions.

Advantageously, the composition is stable if at least 70% by weight, more preferably 80%, and even more preferably 90% by weight of the retinoid content which was incorporated in the composition is still present after thirteen weeks of storage at 40°C and 75% of relative humidity, and 70% by weight, more preferably 80%, and even more preferably 90% of the DHA content which was incorporated in the composition is still present after thirteen weeks of storage at 40°C and 75% of relative humidity. The content of the compounds of the composition according to the present invention can be determined by HPLC (high performance liquid chromatography). By "storage conditions", it should be understood at least thirteen weeks at 40°C and 75% of relative humidity.

Preferably, said emulsion has a pH comprised between 4 and 5, more preferably between 4.1 and 4.9, and

even more preferably between 4.2 and 4.8.

Preferably, the emulsion according to the present invention contains DHA as the only coloring agent.

DHA (dihydroxyacetone) is a colorless sugar (triose carbohydrate) having the chemical formula $C_3H_6O_3$. It can be prepared by the mild oxidation of glycerol, for example with hydrogen peroxide and a ferrous salt as a catalyst. The chemical formula of DHA is illustrated below:

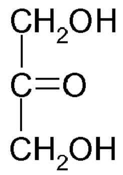

Advantageously, the emulsion according to the present invention comprises from 0.1 to 10 % by weight of DHA relative to the total weight of the emulsion, in particular from 0.5 to 6 % by weight of DHA relative to the total weight of the emulsion, and more particularly from 0.5 to 3 % by weight of DHA relative to the total weight of the emulsion. More advantageously, the emulsion comprises less than 3 % by weight of DHA relative to the total weight of the emulsion, still more advantageously less than 2.5 % by weight of DHA relative to the total weight of the emulsion, in particular less than 2.1 % by weight of DHA relative to the total weight of the emulsion.

As used herein, "retinoid" includes all natural and/or synthetic analogs of Vitamin A or retinal-like compounds which possess the biological activity of Vitamin A in the skin as well as the geometric isomers and stereoisomers of these compounds. The retinoid used in the emulsion according to the invention is selected from retinoic acid or tretinoin, retinaldehydes, retinal, their salts, their esters and the stereoisomers thereof. Examples are retinyl palmitate, retinyl acetate, and stereoisomers such as all transretinoic acid, 9-cis retinoic acid, and 13-cis-retinoic acid.

In addition, the terms "retinol" and "retinoic acid" should be understood to also include the hydrogenated and non-hydrogenated isomers such as β-cis-retinol, didehydroretinol, β-cis-retinoic acid, β-trans-retinoic acid and didehydroretinoic acid.

Typical salts are alkali metal, ammonium and alkylammonium salts. Sodium, potassium, triethanolammonium and ammonium salts are particularly preferred.

As used herein alkylammonium refers to primary, secondary, tertiary and, which is preferred, quaternary alkylammonium, wherein alkyl: in particular is $C_1$-$C_{30}$ alkyl. $C_1$-$C_{30}$ alkyl refers to hydrocarbon radicals having from 1 to 30 carbon atoms that are preferably saturated. Said $C_1$-$C_{30}$ alkyl may be optionally substituted, for example with hydroxy.

The compositions may also contain combinations of one or more of the above mentioned retinoids.

These compounds are well known in the art and are commercially available from a number of sources, e.g., Sigma Chemical Company (St. Louis, Mo.), and

Boerhinger Mannheim (Indianapolis, Ind.). Preferred retinoids comprise retinol, retinyl palmitate, retinyl acetate, retinyl propionate, retinal or mixtures thereof. More preferred are retinol, retinyl palmitate, or mixtures thereof, and even more preferably retinol. 1.

The chemical formula of retinol is illustrated below:

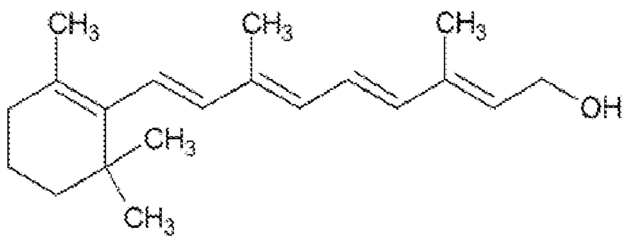

The amount of retinoid in the composition will depend upon the retinoid used and the desired cosmetic effect. Advantageously, the emulsion according to the present invention comprises from 0.01 to 1 % by weight of retinoids relative to the total weight of the emulsion, in particular from 0.01 to 0.5 % by weight of retinoids relative to the total weight of the emulsion, more particularly from 0.01 to 0.3% by weight of retinoids relative to the total weight of the emulsion.

In the sense of the present invention, glycosylated ascorbic acid is intended to mean any natural vitamin C stabilized with a sugar. Advantageously, the sugar is glucose. More advantageously, glycosylated ascorbic acid is acid ascorbic glucoside (AAG) and more advantageously ascorbic acid 2-glucoside (AA2G). AA2G is stable over a wide

range of pH condition, particularly between pH 5 to 7. AA2G is well known in the art and is commercially available from Hayashibara Company. AA2G is known as a whitening agent.

The chemical formula of AA2G is illustrated below:

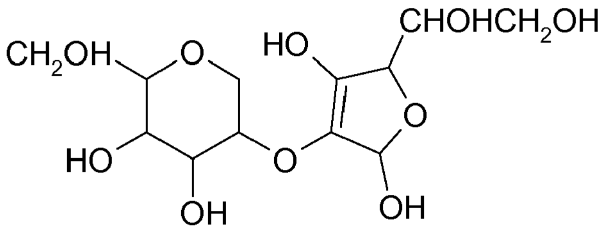

The stabilizing properties seem to be due to the presence of a sugar on the ascorbic acid.

Advantageously, the emulsion according to the present invention comprises from 0.1 to 5 % by weight of a glycosylated ascorbic acid, relative to the total weight of the emulsion, in particular from 0.2 to 3 % by weight of glycosylated ascorbic acid relative to the total weight of the emulsion.

It should be noted that in addition to its stabilizing properties, glycosylated ascorbic acid can be use as a whitening agent. Furthermore, AA2G is also very effective in maintaining the skin resilience and suppressing skin cell damage due to UV.

In one embodiment, the emulsion according to the invention comprises vegetable oil comprising a $C_{18}$ unsaturated fatty acid fraction.

In an advantageous embodiment, the vegetable oil according to the present invention contains more than 40 % of monounsaturated and diunsaturated $C_{18}$ fatty acids.

Advantageously, the vegetable oil is chosen such that the $C_{18}$ unsaturated fatty acid fraction therein is present in a weight ratio of $C_{18}$ unsaturated fatty acid fraction to DHA that is at least about 1:2, preferably at least about 1:1.5, more preferably at least about 1:1.

For example, the vegetable oil may be selected from the group consisting of avocado oil, borage oil, cottonseed oil, sesame oil and a mixture thereof, more advantageously in the group consisting of avocado oil, borage oil and a mixture thereof, still more advantageously it is a mixture of two or more vegetable oils, advantageously a mixture of avocado oil and borage oil.

In particular, borage oil is borage seed oil (Borago Officinalis seed oil) and is sold under the trade name Akostar P and avocado oil (Persea Gratissima) is sold under the trade name refined avocado oil by SICTIA.

The emulsion according to the present invention can comprise from 1 to 10 % by weight of vegetable oil relative to the total weight of the emulsion, advantageously from 2 to 8 % by weight of the vegetable oil relative to the total weight of the emulsion, still more advantageously from 4 to 7% by weight of the vegetable oil relative to the total weight of the emulsion, in particular from 5 to 7% by weight of the vegetable oil relative to the total weight of the emulsion.

In one embodiment, the emulsion according to the present invention is free of color additive.

The term "free of color additive" is intended to mean, in the sense of the present invention that the emulsion according to the present invention does not contain any color additive which: 1) can provide the skin with an immediate tanning appearance before the DHA tanning takes effect, or 2) provides the emulsion with a particular color (e.g., white, off-white, light brown, dark brown, tan, creme, caramel, yellow, orange, blue, green, red, etc.). Therefore in one embodiment, the emulsion according to the present invention is transparent or white. The color additives which are excluded from the emulsion according to the present invention are organic additive, inorganic additive and mica. Non-limiting examples of color additives according to the present invention are described in the International Cosmetic Ingredient Dictionary and Handbook, 10th Edition (2004), which is incorporated into this document by reference. Examples of organic color additives include water soluble dyes, oil soluble dyes, and pigments. Inorganic color additives can produce "earthy" tones which can be used to achieve muted colors. For instance, iron oxides (e.g., red, black, and yellow oxides) in combination with titanium dioxide can be used to achieve a brownish tint. Non-limiting examples of other inorganic color additives include pigments such manganese, ultramarines, titanium dioxides, ferric ferrocyanide, and chromium oxide. Micas use light reflection, refraction, and transmission to exhibit their effects.

In another advantageous embodiment, the emulsion according to the present invention is free of mineral oil and petrolatum.

By formulating the present emulsion as free of mineral oil and petrolatum, the emulsions are non-irritating to sensitive skin, have an enhanced skin-feel when applied, and/or are less occlusive to DHA, retinoids and ascorbic glucosid acid.

In a particular embodiment, the emulsion according to the present invention comprises a gelling agent chosen in the group consisting of a modified starch gelling agent, a branched polysaccharide gelling agent and a mixture thereof. Advantageously, the emulsion according to the present invention comprises a mixture of a modified starch gelling agent and a branched polysaccharide gelling agent.

The modified starch gelling agent used in the emulsion according to the present invention can be for example a crosslinked starch, modified corn starch, hydroxypropylated starch, or a starch phosphate ester. In one embodiment, the modified starch gelling agent is a nonionic polymer. In another embodiment, the modified starch gelling agent has shear thinning properties. In a further embodiment, the modified starch gelling agent is both nonionic and shear thinning. Preferably, the modified starch gelling agent is hydroxypropyl starch phosphate. Hydroxypropyl starch phosphate is commercially available under the name STRUCTURE XL from National Starch and Chemical.

Advantageously, the amount of modified starch gelling agent in the emulsion of the invention varies from 0.1 to 6, more advantageously from 0.5 to 3, still more advantageously of from 1 to 3, weight percent based on the total weight of the emulsion.

According to another embodiment of the invention the emulsion may contain in addition to or in place of the modified starch gelling agent, a branched polysaccharide gelling agent. For example, the branched polysaccharide gelling agent may be selected from xanthan gum and succinoglycan. Preferably, the branched polysaccharide gelling agent is succinoglycan. Succinoglycan is commercially available under the name RHEOZAN SH from Rhodia. The amount of branched polysaccharide gelling agent in the emulsion may vary from 0.01 to 1, preferably from 0.12 to 0.20, weight percent based on the total weight of the emulsion.

The emulsion of the invention may contain additional gelling agents, e.g., based on polyacrylates.

The emulsion of the invention may contain various other optional ingredients known in the art, such as acidifying agents, alkalizing agents, antimicrobial agents, antioxidants, buffering agents, chelating agents, dermotologically active agents, dispersing agents, emollients, emulsifying agents, humectants, fragrances, preservatives, sugars, sunscreen agents, surfactants, suspending agents, thickening agents, and vehicles. Examples of these ingredients are listed below as well as in the ICT Handbook.

Acidifying and alkalizing agents can be added to obtain the desired pH of the emulsion. Examples of acidifying agents included citric acid, lactic acid, glycolic acid, acetic acid, glacial acetic acid, malic acid, and propionic acid. Examples of alkalizing agent include edetol, potassium carbonate, potassium hydroxide, sodium borate, sodium carbonate, sodium citrate, sodium lactate, sodium glycolate, and sodium hydroxide. Other acidifying and alkalizing agents are listed on page 1653 of the ICT handbook.

Antimicrobial agents can be used when the emulsion is to be applied to skin prone to microbial infection, e.g., by bacteria, fungal, or protozoa. Examples of such agents include benzyl alcohol, chlorobutanol, phenylethyl alcohol, phenylmercuric acetate, potassium sorbate, and sorbic acid, benzoic acid, butylparaben, ethylparaben, methylparaben, propylparaben, and sodium benzoate. Other antimicrobial agents are listed on page 1612 of the ICT handbook.

Antioxidants can be used to protect ingredients of the emulsion from oxidizing agents that are included within or come in contact with the emulsion. Examples of antioxidants include water soluble antioxidants such as ascorbic acid, sodium sulfite, metabisulfite, sodium miosulfite, sodium formaldehyde, sulfoxylate, isoascorbic acid, cysteine hydrochloride, 1,4-diazobicyclo-(2,2,2)-octane, and mixtures thereof. Examples of oil-soluble antioxidants include ascorbyl palmitate, butytlated hydroxyanisole, butylated hydroxytoluene, potassium propyl gallate, octyl gallate, dodecyl gallate, phenyl-$\alpha$-napthyl-amine, and tocopherols such as $\alpha$-tocopherol. Other antioxidants are listed on pages 1612-13 of the ICT Handbook.

Buffering agents can be used to maintain an established pH of the emulsion. Examples of buffering agents included calcium acetate, potassium metaphosphate, potassium phosphate monobasic, and tartaric acid. Other buffering agents are listed on page 1612 of the ICT handbook.

Chelating agents can be used to maintain the ionic strength of the emulsion and/or bind to destructive compounds and metals that are included within or come in contact with the emulsion. Examples of chelating agents included edetate dipotassium, edetate disodium, edetic acid, and ethylenediamine tetraacetic acid (EDTA) and its salts (e.g., disodium EDTA). Other chelating agents are listed on page 1626 of the ICT handbook.

Dermotalogically active agents include agents for treating wound healing, inflammation, acne, psoriasis, cutaneous aging, skin cancer, impetigo, herpes, chickenpox, dermatitis, pain, itching, skin irritation. Examples of such dermatalogically active agents include hydrocortisone, dexamethesone, panthenol, phenol, tetracycline hydrochloride, yeast, hexylresorcinol, lamin, kinetin, betamethasone, triamcinolone, fluocinolone, methylprednisolone, dapsone, sulfasalazine, resorcinol, salicylic acid, benzoyl peroxide, erythromycin-benzoyl peroxide, erythromycin, clindamycin, mupirocin, griseofulvin, azoles such as miconazole, econozole, itraconazole, fluconazole, and ketoconazole, ciclopirox, allylamines such as naftifine and terfinafine, acyclovir, famciclovir, valacyclovir, benzocaine, lidocaine, dibucaine, pramoxine hydrochloride, methyl salicylate, camphor, menthol, resocinol, and vitamins such as tocopherol, and tocopherol acetate.

Examples of dispersing and suspending agents include poligeenan, magnesium aluminum silicate xanthan

gum, and silicon dioxide. Other dispersing or suspending agents are listed on page 1612 of the ICT handbook.

Emollients are agents that soften and smooth the skin. Examples of emollients include oils and waxes such as microcrystaline wax, polyethylene, triglyceride esters such as those of castor oil, cocoa butter, safflower oil, corn oil, olive oil, cod liver oil, almond oil, palm oil, squalene, and soybean oil, acetylated monoglycerides, ethoxylated glycerides, fatty acids, alkyl esters of fatty acids, alkenyl esters of fatty acids, fatty alcohols, fatty alcohol ethers, ether-esters, lanolin and derivatives of lanolin, polyhydric alcohol esters, wax esters such as beeswax, vegetable waxes, phospholids, and sterols, isopropyl palmitate or glyceryl stearate. Other emollients are listed on pages 1656-61 of the ICT handbook.

In a particular embodiment, the emulsion according to the present invention comprises an emulsifying agent. Preferably, the emulsion according to the present invention comprises both a gelling agent and an emulsifying agent.

Emulsifying agents can be used for preparing the emulsions of the present invention. Examples of emulsifying agents include Arlacel 165 and methyl gluceth sesquisterate, fatty alcohols, fatty alcohols and alkyl phenols condensed with ethylene oxide. Other emulsifiers are listed on pages 1679-87 of the ICT Handbook. Emulsion stabilizers are listed on pages 1634-35 of the ICT Handbook.

Humectants are agents that typically promote the retention of moisture, e.g., moisturizers. Examples of humectants include sorbitol, glycerin, glycereth 5 lactate, glycereth 7 triacetate, glycereth 7 diisononoate, hexanetriol, glycols such as methyl- propanediol, 1,2-pentanediol, hexylene glycol, and propylene glycol, alkoxylated glucose, D-panthenol and derivatives thereof, and hyaluronic acid. Other humectants are listed on pages 1661-62 of the ICT Handbook.

Siloxane are particularly preferred humectant. Siloxanes that may be used in the present invention include, but are not limited to, dimethicone, cyclomethicone, phenyl trimethicone, phenyl dimethicone, cetyl dimethicone, stearyl dimethicone, amodimethicone, $C_{30-45}$ alkyl dimethicone, $C_{30-45}$ Alkyl Methicone, Cetearyl methicone, dimethicone copolyol, cyclopentasiloxane, cyclohexasiloxane or any combinations thereof.

Examples of fragrances or perfume include peppermint, rose oil, rose water, aloe vera, clove oil, menthol, camphor, eucalyptus oil, and other plant extracts. To eliminate certain odors from emulsions, masking agents may be used. An example of a masking agent includes ethylene brassylate. Other fragrances and masking agents are listed on pages 1639-40 of the ICT Handbook.

Preservatives can be used to protect the emulsion from degradation. Examples of preservatives include phenoxyethanol, methylparaben, benzalkonium chloride, benzethonium chloride, propyl paraben, benzoic acid, benzyl alcohol, and mixtures thereof such as liquipar oil. Other preservatives are listed on pages 1654-55 of the ICT Handbook.

Sugars can be used to improve the results obtained by DHA. Examples of sugars include monosaccharides, disaccharides, and polysaccharides such as glucose, xylose, fructose, reose, ribose, pentose, arabinose, allose, tallose, altrose, mannose, galactose, lactose, sucrose, erythrose, glyceraldehyde, or any combination thereof. However, advantageously the emulsion according to the present invention is free of sugars.

Sunscreen agents are agents typically used to block or reduce the amount of ultraviolet radiation impinging on the skin (e.g., by absorption, scattering, and reflection of the ultraviolet radiation). Segarin, et al., Cosmetics Science and Technology, Chapter VIII, pages 189, et seq. discloses numerous examples of sunscreen agents. Examples of sunscreen agents include both organic compounds and their salts such as, butylmethoxydibenzoyl methane, diethyl hexyl butamido triazone, diethyl amino hydroxybenzoyl hexyl benzoate, ethylhexyl triazone, bis-ethylhexyloxyphenol methoxyphenyl triazine, methylene bis-benzotriazolyl tetramethylbutyl phenol, phenyl benzymidazole sulfonic acid, ethylhexyl salicylate, benzophenone-3, homosalate, octocrylene, avobenzone, and menthyl anthranilate, as well as inorganic particulate materials such as zinc oxide, silica, iron oxide, titanium dioxide, and 2-ethyl-hexyl-p-methoxycinnamate. Other agents are listed on page 1672 of the ICT Handbook.

Generally, the emulsion may contain from 1% to 50%, by weight, of sunscreen agent(s). The exact amounts will vary depending on the sunscreen used and the desired sun-protection factor (SPF).

Surfactants are agents typically used to stabilize emulsion, e.g., used as wetting agents, antifoam agents, emulsifiers, dispersing agents, and penetrates. Examples of surfactants include lapyrium chloride, laureth 4, laureth 9, monoethanolamine, nonoxynol 4, nonoxynol 9, nonoxynol 10, nonoxynol 15, nonoxynol 30, poloxalene, polyoxyl 8, 40, and 50 stearate, polysorbate 20, polysorbate 40, polysorbate 60, polysorbate 65, polysorbate 80, and polysorbate 85, sodium lauryl sulfate, sorbitan, decyl polyglucoside, PPG-5-ceteth-20 and its derivatives. Other surfactants are listed on pages 1672-90 of the ICT Handbook.

Vehicles are often referred to as the base for the cosmetically acceptable carrier, e.g., a fluid that is capable of delivering the other components of the emulsion to the skin with acceptable absorption of those components into the skin. Examples of vehicles include water, e.g., deionized water or demineralized water. In the emulsion according to the present invention, the continuous water phase contains the water soluble agents and the discontinuous oil phase contains the oil soluble agents.

The viscosity of the emulsion may be different dependent on the type of formulation being prepared, e.g., a liquid formulation will have a lower viscosity than a gel formulation. Typically, the viscosity of cream formulations ranges from 5,000 to 150,000 cps. Lower viscosities, such as down to about 500 cps are also suitable for sprays, foams, mists

and the like. Bulking agents may be used to increase the viscosity of the emulsion. An example of a bulking agent is talc. Other bulking agents are listed on page 1625-26 of the ICT Handbook. Other viscosity increasing agents are listed on pages 1693-97 of the ICT Handbook. Viscosity decreasing agents are listed on pages 1692-92 of the ICT Handbook.

Advantageously the emulsion according to the present invention is included in a cosmetic composition or product.

The emulsion according to the present invention can be chosen in the group comprising an oil-in-water emulsion, a water-in-oil emulsion, a silicone-in-water emulsion or a water-in silicone emulsion, and is preferably an oil-in-water emulsion or a water-in-oil emulsion, more preferably an oil-in-water emulsion.

A used herein the term "emulsion" according to the present invention refers to a micellar emulsion system consisting of droplets of an internal phase suspended in a contiguous external phase, a multiphase emulsion system which consists of single micelles of oil and water suspended in a contiguous external phase, or a multivesicular emulsion system which consists of a two- phase, emulsion system which has a multilamellar series of concentric spheres or shells of oil and water phases. Preferably, emulsion according to the present invention is a micellar emulsion system or a multiphase emulsion system, in particular a micellar emulsion system.

The composition of the present invention is suitable for topical application to the skin. The composition may be made into a wide variety of product types that include but are not limited to lotions, creams, gels, sticks, sprays, ointments, pastes, mousses and cosmetics. Preferably, the composition of the present invention is a cream or a gel.

The present invention also concerns a method for the preparation of an emulsion according to present invention, said method comprising the mixing of dihydroxyacetone, a retinoid, and a glycosylated ascorbic acid at a pH comprised between 4 and 5, advantageously between 4.5 and 4.9.

The present invention concerns also a method of coloring the skin and/or unifying skin tone comprising applying the emulsion according to the present invention onto the skin. Preferably, said method enhances the radiance of the complexion of the skin, in particular of the face, so as to give a healthy appearance.

In another embodiment, said method is a skin tanning method.

In another aspect, the present invention concerns the use of a glycosylated ascorbic acid as a stabilizer in a cosmetic composition. More particularly, the present invention concerns the use of a glycosylated ascorbic acid to stabilize an emulsion comprising DHA and a retinoid, in particular retinol. 1.

In a further aspect, the present invention concerns the use of a glycosylated ascorbic acid as a pH stabilizer in a cosmetic composition and more particularly, the use of a glycosylated ascorbic acid to stabilize the pH of a composition comprising DHA and a retinoid, in particular retinol.

In another aspect, the emulsion of the present invention is useful to prevent or to combat the effects of skin aging. The latter comprise effects such as the appearance of fine lines, fine wrinkling, wrinkling, loss of skin firmness, skin tightening and suppleness. The effects of skin aging are due to aging as such, but also to aging of the skin caused by external factors such as exposure to environmental factors such as exposure to sunlight, wind, atmospheric pollutants and the like, or a combination of these factors.

Preferably, the emulsion of the present invention is active on the superficial layer of the skin, and more preferably the composition of the present invention is active only on the epidermis.

Thus, the present invention further concerns a method of preventing or combating cutaneous ageing, in particular the appearance on the skin of fine lines, fine wrinkling and/or wrinkling, and/or the loss of skin firmness, skin tightening and suppleness, said method comprising a step of applying the emulsion according to the invention to the skin.

In a preferred embodiment, the use of an emulsion according to the invention permits to tan the skin and to prevent or to combat cutaneous aging.

Furthermore, the emulsion of the present invention is useful to prevent or to combat skin ageing and/or certain skin disorders, such as acne or disorders of keratinization or of healing.

Thus, the present invention also concerns an emulsion as described above to prevent or combat skin ageing and/or skin disorders, such as acne or disorders of keratinization or of healing.

The following non-limiting examples further illustrate the invention.

### Example 1: Emulsions according to the present invention

Table 1 illustrates one emulsion (composition 1) according to the present invention (ingredients and weight %), and the preferred ranges of the compounds to prepare an emulsion according to the invention.

Table 1

| INCI Name | % wt | Composition 1 (% wt) |
|---|---|---|
| Water | qsp 100 | qsp 100 |

(continued)

| INCI Name | % wt | Composition 1 (% wt) |
|---|---|---|
| Hydroxypropyl Starch Phosphate | 0.1-5 | 0.5-2 |
| Succinoglycan | 0.05-5 | 0.1-0.5 |
| Glycerin | 0.5-10 | 0.8-1.5 |
| Methylparaben | 0.1-0.3 | 0.15-0.25 |
| Phenoxyethanol | 0.1-0.8 | 0.4-0.8 |
| Sorbitol | 0.5-10 | 2-4 |
| Disodium EDTA | 0.05-0.3 | 0.08-0.12 |
| Polyacrylate-13 ; Polyisobutene ; Polysorbate20 ; water. | 0.1-3 | 0.2-0.5 |
| Arachidyl alcohol; Arachidyl glucoside; Behenyl alcohol. | 0.5-5 | 2-4 |
| PEG-100 Stearate ; Glyceryl Stearate. | 0.5-5 | 1-2 |
| Ethylhexyl Stearate | 1.0-20.0 | 8-12 |
| BHT | 0.01-1 | 0.8-1.2 |
| Cetyl alcohol | 0.1-3 | 0.3-1 |
| Propylparaben | 0.1-0.3 | 0.1-0.3 |
| Hydrogenated Palm Glycerides Citrate; Olus Oil; Tocopherol | 0.05-0.3 | 0.1-0.2 |
| Cyclohexasiloxane, Cyclopentasiloxane | 0.5-5 | 1-3 |
| Dihydroxyacetone | 0.1-10 | 0.5-3 |
| AA2G | 0.1-5 | 0.2-3 |
| Sodium Hydroxide | 0.1-2 | 0.01-0.6 |
| Ascorbic Acid | 0.01-1 | 0.03-0.1 |
| Retinol | 0.01-1 | 0.01-0.3 |
| Total | 100.00 | 100.00 |

The process for preparing the emulsion of Table 1 was as follows:

In a main vessel, water is mixed with the hydroxypropyl starch phosphate. The mixture is then heated at 75°C. The mixture is added to a premix of glycerin and succinoglycan. Then, methylparaben, phenoxyethanol, sorbitol and EDTA are added.

Around 60°C, the (polyacrylate-13, polyisobutène, polysorbate20, water) is added.

To prepare the oil phase, (Arachidyl alcohol, Arachidyl glucoside; Behenyl alcohol.), the Ethylhexylstearate, the Cetyl alcohol, the (PEG-100 Stearate, Glyceryl Stearate) and the (Cyclohexasilosane, Cyclopentasiloxane) were mixed.

Propylparaben and the (Hydrogenated Palm Glycerides citrate, Olus Oil, Tocopherol) and BHT were added. The emulsion is prepared at 75°C, and then cooled down below 30°C. DHA is added and pH is adjusted by a premix of water/AA2G/sodium hydroxide.

Finally, ascorbic acid and retinol are added.

## Example 2. Stability study Retinol + DHA

Table 2

| Formula | All trans retinol content stability in % by weight after 6 weeks of storage at 50°C | DHA content stability in % after 6 weeks of storage at 50°C | pH after 6 weeks of storage at 50°C |
|---|---|---|---|
| Comparative example 1: Composition 1 without AA2G pH = 5.01 | 83 | 68 | 3.9 |
| Comparative example 2: Composition 1 without AA2G pH = 5.7 | 84 | 59 | 4 |
| Comparative example 3: Composition 1 without AA2G and DHA pH=6 | 92 | | |
| Comparative example 4: Composition 1 without AA2G + pH pH = 4.17 | 55 | 81 | 3.6 |
| Comparative example 5: Composition 1 without AA2G pH = 4.7 | 69 | 74 | 3.9 |
| Comparative example 6: Composition 1 without AA2G and with citrate buffer pH = 4.7 | 90 | 21 | 4.3 |
| Comparative example 7: Composition 1 without AA2G and with phosphate buffer (pH = 4.7) | 85 | 57 | 3.7 |
| **Composition 1 (pH = 4.7)** | **93** | **72** | **4.1** |

Protocol

The composition 1 of example 1 was tested for the content stability of DHA and retinol and comparative examples of emulsions similar to the composition 1 but at different pH (by the use of sodium hydroxide), without AA2G, with or without DHA, and with or without different buffers were prepared.

The content of DHA and retinol was determined by HPLC (high performance liquid chromatography) at t=0 and after 6 weeks of storage at 50°C.

The content stability was calculated as follows:

content stability % = (content after six weeks of storage at 50°C x 100) / (content at t=0).

In the cosmetic field, accelerated ageing of the composition is usually obtained after 13 weeks of storage at

40°C and 75% of relative humidity. However, a more accelerate ageing of the composition can be obtained after six weeks of storage at 50°C.

The pH of the composition was also evaluated.

Results

Table 1 shows the percentage of content stability of both retinol and DHA for each of the emulsions.

These results clearly indicate that the best stability for both DHA and retinol is obtained when the composition is prepared at pH 4.7, and that stability can be improved by adding AA2G.

This is confirmed by the results obtained after 13 weeks of storage of the composition 1 at 40°C and 75% of relative humidity (pH = 4.3, 94.3% of retinol content stability, and 72.5% of DHA content stability).

Moreover, the use of citrate buffer or phosphate buffer in a composition comprising retinol and DHA without AA2G dramatically decreases the stability of DHA to 21% and 57% respectively, thus underlying the fact that glycosylated ascorbic acid acts as a specific pH stabilizer.

Furthermore, the results also indicate that the addition of AA2G in a composition prepared at a pH of 4.7 permits to stabilize the pH to 4.2 after 6 weeks of storage at 50°C or to 4.31 after 13 weeks of storage at 40°C and 75% of relative humidity whereas it decreases to 3.85 when no AA2G is used after 6 weeks of storage at 50°C.

In conclusion, the inventors of the present invention clearly demonstrated that the addition of AA2G to a composition comprising retinol and DHA prepared at a pH of 4.7 permits to stabilize both of the two compounds and also permits to stabilize the pH of said composition.

## Claims

**1.** An emulsion comprising:

a) dihydroxyacetone,
b) a retinoid, and
c) a glycosylated ascorbic acid.

**2.** The emulsion according to claim 1, said emulsion having a pH comprised between 4 and 5.

**3.** The emulsion according to claim 2, said emulsion having a pH comprised between 4.1 and 4.8.

**4.** The emulsion according to any of claims 1 to 3, wherein it comprises from 0.1 to 10% by weight of DHA relative to the total weight of the emulsion, advantageously from 0.5 to 6 % weight of DHA relative to the total weight of the emulsion.

**5.** The emulsion according to any of claims 1 to 4, wherein it comprises from 0.01 to 1 % by weight of retinoids relative to the total weight of the emulsion, advantageously from 0.01 to 0.5 % by weight of retinoids relative to the total weight of the emulsion, more particularly from 0.01 to 0.3% by weight of retinoids relative to the total weight of the emulsion.

**6.** The emulsion according to any of claims 1 to 5, wherein it comprises from 0.1 to 5 % by weight of glycosylated ascorbic acid relative to the total weight of the emulsion, advantageously from 0.2 to 3 % by weight of glycosylated ascorbic acid relative to the total weight of the emulsion.

**7.** The emulsion according to any one of claims 1 to 6, wherein it comprises a gelling agent selected from the group consisting of a modified starch gelling agent, a branched polysaccharide gelling agent and a mixture thereof.

**8.** The emulsion according to any one of claims 1 to 7, wherein said retinoid is retinol.

**9.** A method of coloring the skin and/or unifying skin tone comprising applying the emulsion of any one of claims 1 to 8 onto the skin.

**10.** The method according to claim 9, to enhance the radiance of the complexion of the skin, in particular of the face, so as to give a healthy appearance.

**11.** A method of preventing or combating the appearance on the skin of fine lines, fine wrinkling and/or wrinkling, and/or the loss of skin firmness, skin tightening and suppleness, said method comprising a step of applying the emulsion of any one of claims 1 to 8 to the skin.

**12.** A method for the preparation of an emulsion according to any of claims 1 to 8, said method comprising the mixing of dihydroxyacetone, a retinoid, and a glycosylated ascorbic acid at a pH comprised between 4.5 and 4.9.

**13.** Use of a glycosylated ascorbic acid, to stabilize an emulsion comprising DHA and a retinoid.

**14.** The emulsion according to any of claims 1 to 8, to prevent or combat skin ageing and/or skin disorders, such as acne or disorders of keratinization or of healing.

Europäisches Patentamt
European Patent Office
Office européen des brevets

# EUROPEAN SEARCH REPORT

Application Number
EP 08 30 5675

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 473 026 A (BEIERSDORF AG [DE]) 3 November 2004 (2004-11-03) * paragraphs [0028] - [0035]; example 15 * ----- | 1-14 | INV. A61K8/35 A61K8/67 A61Q19/04 |
| Y | WO 2007/066311 A (PROCTER & GAMBLE [US]; TANAKA SHUHEI [JP]; MORI KIYOAKI [JP]) 14 June 2007 (2007-06-14) * page 17, line 8 - line 12; claims 8-10; example 1 * ----- | 1-14 | |
| Y | WO 2006/022174 A (HAYASHIBARA BIOCHEM LAB [JP]; KUBOTA MICHIO [JP]; FUKUSHIMA HIDEKI [JP) 2 March 2006 (2006-03-02) * experiment 1 * * example 23 * ----- | 1-14 | |
| D,Y | WO 2006/009987 A (E L MANAGEMENT CORP [US]; LENTINI PETER J [US]; BEVACQUA ANDREW [US];) 26 January 2006 (2006-01-26) * claims 1,3; table 1 * ----- | 1-14 | TECHNICAL FIELDS SEARCHED (IPC) |
| D,Y | WO 02/072039 A (PENTAPHARM LTD [CH]; YAMAMOTO TAKASHI [JP]; NAKAYAMA HIROKI [JP]) 19 September 2002 (2002-09-19) * claims 1,7,8 * ----- | 1-14 | A61K A61Q |
| A | EP 0 754 449 A (OREAL [FR]) 22 January 1997 (1997-01-22) * page 2, line 39 - line 57 * ----- | | |

The present search report has been drawn up for all claims

| Place of search | Date of completion of the search | Examiner |
|---|---|---|
| Munich | 31 March 2009 | Baumeister, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

7

EPO FORM 1503 03.82 (P04C01)

## ANNEX TO THE EUROPEAN SEARCH REPORT ON EUROPEAN PATENT APPLICATION NO. EP 08 30 5675

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2009

| Patent document cited in search report | Publication date | Patent family member(s) | Publication date |
|---|---|---|---|
| EP 1473026 A | 03-11-2004 | DE 10319373 A1 | 18-11-2004 |
| WO 2007066311 A | 14-06-2007 | CN 101325942 A | 17-12-2008 |
| | | EP 1957035 A1 | 20-08-2008 |
| | | KR 20080067373 A | 18-07-2008 |
| | | US 2007134173 A1 | 14-06-2007 |
| WO 2006022174 A | 02-03-2006 | NONE | |
| WO 2006009987 A | 26-01-2006 | AU 2005265249 A1 | 26-01-2006 |
| | | CA 2571553 A1 | 26-01-2006 |
| | | EP 1773366 A2 | 18-04-2007 |
| | | JP 2008503580 T | 07-02-2008 |
| | | KR 20070024730 A | 02-03-2007 |
| WO 02072039 A | 19-09-2002 | NONE | |
| EP 0754449 A | 22-01-1997 | CN 1147936 A | 23-04-1997 |
| | | DE 69600106 D1 | 02-01-1998 |
| | | DE 69600106 T2 | 26-03-1998 |
| | | ES 2112076 T3 | 16-03-1998 |
| | | FR 2736829 A1 | 24-01-1997 |
| | | JP 3004206 B2 | 31-01-2000 |
| | | JP 9030921 A | 04-02-1997 |
| | | US 5882658 A | 16-03-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02072039 A
- WO 2006009987 A


### Non-patent literature cited in the description


- International Cosmetic Ingredient Dictionary and Handbook. 2004
- **Segarin et al.** Cosmetics Science and Technology. 189